# EUROPEAN PATENT APPLICATION

(11) **EP 3 730 214 A1**
(43) Date of publication of application: **28.10.2020**
(21) Application number: 19170969.0
(22) Date of filing: 24.04.2019
(51) Int. Cl.: B01L 3/00, G01N 33/49, G01N 15/14, G01N 1/40

(54) **METHOD AND DEVICE FOR THERMAL INHOMOGENEITY SEPARATION**

(71) Applicant: AcouSort AB, 223 81 Lund (SE)
(72) Inventor: AUGUSTSSON, Per, 417 49 Göteborg (SE)
(74) Representative: Brann AB

(57) **Abstract**

1. A method of performing an acoustophoretic operation comprises the steps of: i. providing a fluid, ii. positioning the fluid in a microfluidic cavity, iii. subjecting at least one portion of the fluid, in the microfluidic cavity, to an acoustic wave, and iv. providing, in at least one first region of the at least one portion, a thermal inhomogeneity whereby the temperature of the fluid in the at least one first region differs from the temperature of the fluid in at least one second region of the remainder of the at least one portion. A microfluidic system is also disclosed.

## Description

### Technical field

The technology proposed herein relates generally to the field of acoustofluidic devices and systems and methods of handling or separating particles or cells in such devices. More particularly the technology proposed herein concerns the use of a purposefully caused thermal inhomogeneity, such as a thermal gradient, for further influencing the behavior of, and effects on, fluids and/or particles or molecules in fluids when subjected to an acoustic wave.

### Background

Acoustofluidic devices and systems include microfluidic acoustofluidic chips comprising acoustofluidic, e.g. microfluidic, cavities such as channels in which a sample fluid can be processed by performing an acoustofluidic operation on the sample liquid. During the acoustofluidic operation ultrasound energy is supplied to the acoustofluidic device including any microfluidic channel or cavity to affect the sample fluid and any particles suspended in the sample fluid. The acoustofluidic operation may be one of separation, i.e. affecting movement of the particles in the sample fluid so that different particles travel different distances in the fluid as a function of how each particles is affected by the ultrasound energy being supplied to the acoustofluidic device. By suitable design of the microfluidic channels or cavities, together with suitable selection of laminar flow of the sample or no-flow conditions, different particles can be moved to different positions and thereafter separated from each other. Other acoustofluidic operations involve driving particles in the sample fluid towards a position, such as the center or walls, of the microfluidic channels or cavities.

As regards separation the different types of fluids and particles may present obstacles to a successful and/or efficient separation. For example, if a desired particle has similar acoustic contrast, in relation to the suspending fluid, or other, non-desired particles, the separation may fail as both types of particles would be affected similarly by the ultrasound energy and the associated acoustic field that is present in the cavity or channel.

If the particles to be separated or otherwise affected by the acoustic field are small, i.e. of sub-micron size, then the force exerted on them due to the acoustic field can be insufficient to move them a sufficient distance to allow separation within a reasonable time-frame.

The separation of sub-micron particles may further be hampered by acoustic streaming, i.e. where the sound field generates slow but steady streaming rolls, e.g. circular flow patterns, in the cross section that is transverse to the length of the channel. Streaming emanates from the thin viscous boundary layers at the top and bottom of the channel and imposes a drag force on particles that competes with the radiative forces acting on them such that they become mixed in the streaming rolls. As the radiative force is proportional to the cube of the radius of the particle, and as the speed of the particle is proportional to the square of the radius of the particle, large particles will move sufficiently fast so as to surpass the velocity of the streaming, whereas smaller particles will move too slow and be become mixed in the streaming rolls.

A further acoustophoretic operation of interest is mixing of different fluids and or mixing particles or molecules in a fluid. Such mixing takes a long time if allowed to proceed by diffusion only. The rate of mixing may be increased by subjecting the fluids to an acoustic wave, however there may still be applications where it would be advantageous if the rate of mixing were increased even further.

There is accordingly a need for further methods of performing acoustophoretic operations which are more efficient and/or more discriminatory.

### Object of the Technology

Accordingly it is an object of the technology proposed herein to provide devices and methods for performing acoustophoretic operations with an increased efficiency and/or increased discriminatory effect.

### Summary

At least one of the abovementioned objects, or at least one of the further objects which will become evident from the below description, are according to a first aspect of the technology proposed herein obtained by a method of performing an acoustophoretic operation comprising the steps of:
i. providing a fluid,
ii. positioning the fluid in a microfluidic cavity,
iii. subjecting at least one portion of the fluid, in the microfluidic cavity, to an acoustic wave, and
iv. providing, in at least one first region of the at least one portion, a thermal inhomogeneity whereby the temperature of the fluid in the at least one first region differs from the temperature of the fluid in at least one second region of the remainder of the at least one portion.

Accordingly the technology proposed herein is based on the discovery that a thermal inhomogeneity can be used in combination with an acoustic wave for performing acoustophoretic operations. This is surprising in that a strict temperature control is generally desired when performing acoustophoretic operations. Instead here the thermal flows caused by a thermal inhomogeneity are specifically used to modify or supplement the effects of the acoustic wave on the fluid and any particles in the fluid.

The acoustophoretic operation may be any of mixing of fluids with or without particles and/or molecules, sorting of particles and/or molecules, separation of particles and/or molecules, etc.

The fluid may be plasma, water, urine, yeast cell broth, cell culture medium, saline solutions, phosphate buffered saline, interstitial fluid, milk plasma etc. Where the fluid comprises particles the particles may comprise red blood cells, white blood cells, platelets, cancer cells, circulating tumor cells, bacterial cells, viruses, yeast cells, fat cells, exosomes, extracellular vesicles, microvesicles, lipoproteins, dust particles, silica particles and polymer particles. The fluid may be positioned in the cavity by pumping, by pressure, by suction, by the action of electrical fields, by gravity, and/or by capillary action.

The microfluidic cavity may be closed to the environment. The microfluidic cavity is preferably a channel having a square or rectangular cross section. The microfluidic cavity may for example have a cross sectional width of 1 to 10 times, such as 1 to 2 times the cross sectional height. In this case the length of the microfluidic cavity is at least the same as the width, but preferably many times longer. The width may for example be from 0.15 mm to 1 mm. The height may for example be from 0.1 mm to 5 mm.
The microfluidic cavity may be formed in a substrate. The substrate may be made of silicon but may be made of polymeric material such as plastic, or alternatively glass. Also other materials such as ceramics and metals are possible. These materials are cheap and therefore suitable for performing the optical or electrical measurements in the field on in a point of care setting, as disposable consumables.
The substrate may be planar, such as a chip, or alternatively the substrate may be formed as a capillary.

Ultrasound energy, for causing the acoustic wave may be transferred to the substrate from at least one ultrasound transducer connected to the substrate. The ultrasound transducer may be connected to the substrate by adhesives, or by being positioned so that they contact each other.
The at least one portion of the fluid may be a minor portion of the fluid or may comprise all of the fluid in the cavity.

The acoustic wave may preferably be an acoustic standing wave. The acoustic standing wave is correlated with a resonance frequency of the microfluidic cavity. Resonance frequencies of the microfluidic cavity are dependent on the dimensions because, in order for a standing wave to form the wavelength A of the wave, which wavelength is inversely proportional to the frequency, must be nλ/2 where n is a positive integer. Accordingly the term resonance frequency is to be understood to comprise any frequency in which a standing wave may form in the cavity.

The thermal inhomogeneity may be provided in one first region or several first regions of the at least one portion. The thermal inhomogeneity may further be provided throughout the first region. The thermal inhomogeneity may be stepwise continuous, i.e. as a gradient, or stepwise.

The temperature of the fluid in the at least one first region may differ from the temperature of the fluid in at least one second region of the remainder of the at least one portion by at least 0.1°C.

The fluid may be caused to flow through the cavity. The flow may be generated by pumping, by pressure, by suction, by the action of electrical fields, by gravity, and by capillary action. The cavity may be fluidly connected to an inlet, through which the fluid is introduced into the cavity, and fluidly connected to an outlet, through which the fluid leaves the cavity. More than one outlet may be fluidly connected to the cavity in order to lead off different parts of the fluid to different outlets.

By heating the fluid in the at least one first region this fluid obtains an acoustic contrast (as it has a lower compressibility) in relation to the surrounding non-heated fluid. The fluid in the at least one first region is thus rapidly forced, by the acoustic wave, towards a pressure node of the acoustic wave. This may be used to move a particle within the at least one first region with the first region. The particles may be acoustically pre-focused by a 2D acoustic field before entering the first region. One or more outlets may be provided for receiving and discharging the fluid and the particle.
Simulations show that by focused laser irradiation in a spot inside a microfluidic cavity a temperature gradient will rapidly form which can drive a local flow of ∼10 mm/s. Thermal diffusion is very fast at the microscale (∼100 µm in 100 ms) and thermal inhomogeneity forms at the millisecond scale. Cells, bacteria or biological nanoparticles that flow through such a cavity can thus be selectively transported on demand by a localized short burst of sound and light without affecting nearby particles.

Alternatively the thermal inhomogeneity, in form of a thermal gradient, may be used to provide steady flow which can be directed counter to the focusing direction of particles in the acoustic field. Thereby particles will end up in different and stationary positions depending on their acoustic contrast and size while flowing through the cavity.

In the preferred embodiments of the method according to the first aspect of the technology proposed herein the temperature of the fluid in the at least one first region differs by at least 0.1°C from the temperature of the fluid in the at least one second region of the at least one portion. The higher the difference in temperature, the greater the effect of the thermal inhomogeneity on the acoustophoretic operation. Preferably the temperature of the fluid in the at least one first region differs by at least 0.1 °C, such as at least 0.5 °C, 1 °C or 2 °C, from the temperature of the fluid in the at least one second region of the at least one portion

In some embodiments of the method according to the first aspect of the technology proposed herein the thermal inhomogeneity comprises a thermal gradient throughout at least the at least one first region. Such a thermal inhomogeneity may be obtained by heating the at least one first region, or heating a fluid volume or part of the microfluidic cavity adjacent the at least one first region. On or more walls of the microfluidic cavity may for example be heated, and in turn heat the fluid, by providing resistive electrical leads or electrodes on the one or more walls. This is advantageous as it allows the heating device, i.e. the resistive electrical leads or electrodes, to be integrated with the microfluidic cavity. If the acoustophoretic operation to be performed is mixing then it is preferred that the direction of the thermal gradient and the direction of the acoustic wave are different, and preferably, orthogonal to each other.

In some embodiments of the method according to the first aspect of the technology proposed herein the thermal inhomogeneity is effected by directing electromagnetic radiation into the at least one first region. This is advantageous in that it allows easier direction and placement of the thermal inhomogeneity within the microfluidic cavity. Heat is transferred to the fluid by light absorption which can be altered by adding solute dye molecules to the fluid that absorbs light at a specific wavelength. The electromagnetic radiation may for example be directed, and preferably focused, into the fluid to heat the fluid. Alternatively the electromagnetic radiation may be directed towards one or more walls of the microfluidic cavity to heat the one or more walls, which in turn heats the fluid. The electromagnetic radiation may be scanned throughout the cavity to heat the fluid in the at least one first region or may be scanned and pulsed to form a desired pattern or a spatial light modulator can be used to project a programmed pattern of electromagnetic radiation.
In some embodiments of the method according to the first aspect of the technology proposed herein wavelength of the electromagnetic radiation is configured to heat the fluid by absorption. Here the wavelength of the electromagnetic radiation is chosen based on the absorption characteristics of the fluid so that the fluid absorbs the electromagnetic radiation.

In some embodiments of the method according to the first aspect of the technology proposed herein the fluid comprises a particle or molecule and the wavelength of the electromagnetic radiation is configured to heat the particle or molecule by absorption, and wherein the absorption coefficient of the particle or molecule for the electromagnetic radiation differs from the absorption coefficient of the fluid for the electromagnetic radiation. This is advantageous in that this localizes the thermal inhomogeneity to the vicinity of the particle. As the particle is heated the fluid surrounding the particle is also heated. This provides a thermal gradient around the particle. The heated particle and heated surrounding fluid then are affected by a much higher force from the acoustic wave compared to a particle that is not heated and accordingly not surrounded by heated fluid. Further heated surrounding fluid has a lower viscosity, thus reducing drag on the particle As an example hemoglobin absorbs at 420 nm. Thus light of 420 nm wavelength may be used to heat hemoglobin, as well as red blood cells containing hemoglobin.

In some embodiments of the method according to the first aspect of the technology proposed herein the wavelength of the electromagnetic radiation comprises IR-light and visible light. Such electromagnetic radiation can be readily supplied by LEDs and lasers.

In preferred embodiments of the method according to the first aspect of the technology proposed herein the acoustic wave is an acoustic standing wave.

At least one of the abovementioned objects, or at least one of the further objects which will become evident from the below description, are according to a second aspect, corresponding to the first aspect, of the technology proposed herein achieved by a microfluidic system for performing an acoustophoretic operation, the system comprising:
a substrate with a microfluidic cavity formed in the substrate, the microfluidic cavity having an inlet for allowing a fluid into the microfluidic cavity,
an ultrasound transducer connected to the substrate for generating an acoustic wave in at least one portion of the fluid in the microfluidic cavity,
a drive circuit connected to the ultrasound transducer and configured to drive the ultrasound transducer to provide the acoustic wave, and
a thermal device configured to provide, in at least one first region of the at least one portion, a thermal inhomogeneity whereby the temperature of the fluid in the at least one first region differs from the temperature of the fluid in at least one second region of the remainder of the at least one portion.

The microfluidic system may further comprise a container for storing the fluid, a pump or other device for causing the fluid to enter the cavity, a temperature control device for heating or cooling the substrate as needed, and receptacles for receiving the fluid from the cavity.

As above the substrate may be made of silicon, polymers such as plastic, or glass.

As above the microfluidic cavity is preferably elongated.

The ultrasound transducer is preferably a piezoelectric actuator. The ultrasound transducer may be connected to the substrate. The acoustic wave is generated by the vibrations in the ultrasound transducer being transferred to the substrate and causing the walls of the cavity to vibrate.

The drive circuit may comprise a function generator electrically connected to the ultrasound transducer. To provide a sufficiently strong acoustic field, the signal to the transducer can be amplified by an amplification circuit.

As above the temperature of the fluid in the at least one first region differs from the temperature of the fluid in at least one second region of the remainder of the at least one portion by at least 0.1 °C

As an alternative to heating devices cooling devices, or one or more heating devices and one or more cooling devices may be used to create the thermal inhomogeneity.

In preferred embodiments of the system according to the second aspect of the technology proposed herein the thermal device comprises a LED and/or laser arranged, for example in an optical seupt that may include lenses, for irradiating the at least one first region of the at least one portion. This is advantageous in that it allows easier direction and placement of the thermal inhomogeneity within the microfluidic cavity.

In further embodiments of the system according to the second aspect of the technology proposed herein the thermal device further comprises a heating device for heating the substrate for causing a thermal gradient throughout the substrate and throughout the microfluidic cavity. This allows the creation of thermal gradient throughout the substrate.

In further embodiments of the system according to the second aspect of the technology proposed herein the thermal device further comprises a heating device positioned within the microfluidic cavity or on an inner wall of the microfluidic cavity for heating the fluid in the microfluidic cavity. Alternatively, as above, the heating device may comprise resistive electrical leads or electrodes on one or more walls of the microfluidic cavity.

In further embodiments of the system according to the second aspect of the technology proposed herein the microfluidic system further comprises:
a detector configured for detecting that a particle of interest is present in the at least one first region and for outputting a signal when the particle of interest is present in the at least one first region, and
a relay device configured for receiving the signal from the detector and for energizing the drive circuit and the thermal device for causing movement of the fluid in the at least one first region and the particle of interest.
This allows the separation or sorting of the particle of interest even where the particle of interest is too small (i.e. of submicron size) that it would travel too slowly, e.g. slower than the acoustic streaming, to be separated by the acoustic wave alone.
The detector may for example detect the particle of interest by fluorescence or impedance or any other method of detecting particles in a fluid. The detector may for example comprise a camera, a spectrophotometer, and/or a microscope.

In preferred embodiments of the system according to the second aspect of the technology proposed herein the acoustic wave is an acoustic standing wave.

At least one of the abovementioned objects, or at least one of the further objects which will become evident from the below description, are according to a third aspect, corresponding to the first aspect, of the technology proposed herein achieved by the use of a thermal inhomogeneity provided in at least one first region of at least one portion of a fluid a microfluidic cavity in combination with an acoustic wave provided in the fluid for performing an acoustophoretic operation in or on the fluid.
Details of embodiments of the method according to the first aspect of the technology proposed herein are also applicable to the use according to the third aspect of the technology proposed herein.

Further advantages with and features of the technology proposed herein will be apparent from the other dependent claims as well as from the following detailed description of preferred embodiments.

### Brief description of the drawings and detailed description

A more complete understanding of the abovementioned and other features and advantages of the technology proposed herein will be apparent from the following detailed description of preferred embodiments in conjunction with the appended drawings, wherein:
- Fig. 1A: shows acoustic streaming in a cross section of microfluidic channel, the acoustic streaming forming rotating flows as indicated by the small arrows, the streaming being caused by the harmonically oscillating 1st order acoustic velocity field, indicated by black arrows, as the velocity approaches zero at the boundaries (walls) of the channel,
- Fig. 1B: shows a simulation of the velocity of the fluid in a similar cross section under the influence of both a standing wave (acoustic velocity field) and a temperature gradient applied to the substrate in which the microfluidic channel is positioned,
- Fig. 1C: shows a system for providing an acoustic field in combination with a temperature gradient,
- Fig. 2A: shows a simulation of the velocity of fluid under the influence of both a standing wave (acoustic velocity field) and a local thermal inhomogeneity,
- Fig. 2B: shows a system for providing an acoustic field in combination with a local thermal inhomogeneity, and
- Fig. 3A-B: shows a system for providing an acoustic field in combination with a local thermal inhomogeneity spatially located to a particle absorbing wide-field laser radiation.

In the figures and the description the same reference numeral is used to refer to the same feature. A ' added to a reference numeral indicates that the feature so referenced has a similar function, structure or significance as the feature carrying the reference numeral without the ', however not being identical with this feature.

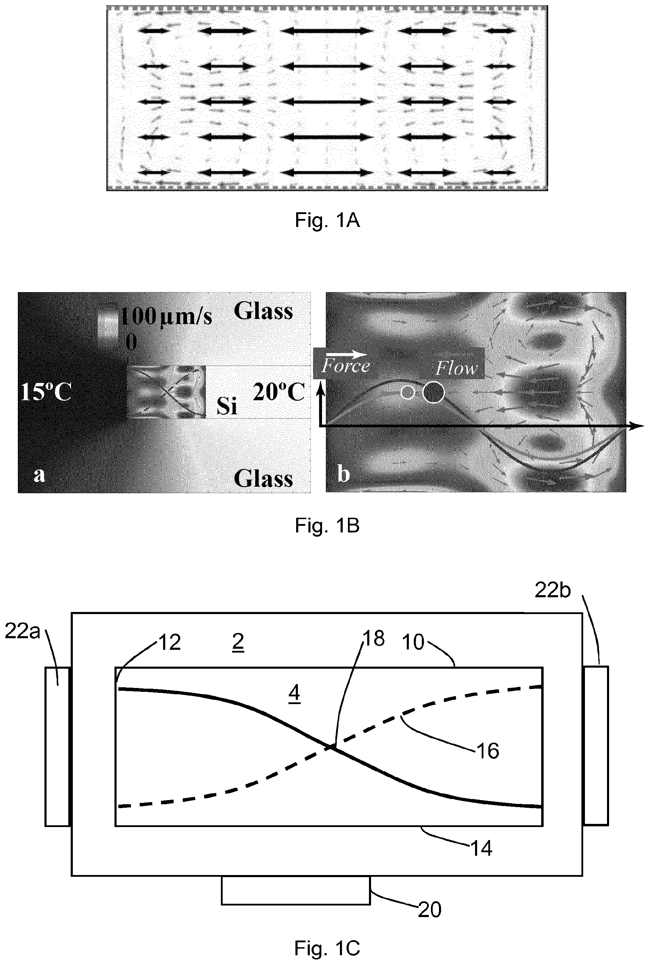

Fig. 1A shows acoustic streaming in a cross section of microfluidic channel, the acoustic streaming forming rotating flows as indicated by the small arrows, the streaming being caused by the harmonically oscillating 1st order acoustic velocity field, indicated by black arrows, as the velocity approaches zero at the boundaries (walls) of the channel. These rotating flows, four of which are shown in Fig. 1A may be used purposefully, to increase the mixing of the fluid in the cavity, but they may also impair the results when it is desired to move smaller particles where the velocity of the particles become lower than the velocity of the rotating flows.

Fig. 1B shows a simulation of the velocity of the fluid in a similar cross section under the influence of both a standing wave (acoustic velocity field) and a temperature gradient applied to the substrate in which the microfluidic channel is positioned. Here the mixing effect of the rotating flows from the streaming is increased by adding a thermal inhomogeneity in the form of a thermal gradient across the glass/silicon substrate in which the microfluidic channel is fabricated, see panel a. The thermal gradient in the substrate results in that the left and right side wall of the channel have different temperatures as indicated by the heat map, where the left side wall has a temperature of about 15 °C and the right sidewall has a temperature of about 20 °C. This thermal gradient causes a thermal flow of the fluid in the channel. As seen in panel b the velocity of the fluid is affected here a result of a combination of the acoustic streaming and the thermal flow. The combination results in a less ordered flow when compared to Fig 1A, and therefore results in a more efficient mixing of the fluid as well as any particles or molecules in the fluid. Theoretical calculations show that up to a 10-fold or larger increase in the mixing efficiency or flow velocities over the mixing obtained by acoustic streaming alone can be obtained. Alternatively a thermal gradient may be used to attempt to cancel out the effects on of acoustic streaming on particles in the fluid 4.

Fig. 1C shows a system for providing an acoustic field in combination with a temperature gradient. A channel 10 is shown in cross section in a substrate 2. A fluid 4 is provided in the channel 10. The channel 10 has opposing side walls, one of which is designated the reference numeral 12, and opposing bottom and ceiling, one of which is designated the reference numeral 14. An acoustic standing wave 16 has a pressure node 18 in the center of the channel 10. The acoustic standing wave 16 is caused by an ultrasound transducer 20 attached to the bottom of the substrate 2 and energized by a drive circuit or function generator (not shown) at a frequency corresponding to the standing wave 16. In order to create a temperature gradient throughout the fluid 4 in the channel first and second heating devices, such as resistive heating devices, 22a and 22b are attached to the outer sidewalls of the substrate 2. By selectively energizing the heating devices 22a and 22b to different temperatures the substrate 2, including the fluid 4 in the channel 10, will experience a temperature gradient. As an alternative to heating devices also cooling devices can be used. Thus one of 22a and 22b may be a cooling device to obtain even larger thermal gradients. The system shown in Fig. 1C may thus be used to efficiently mix the fluid 4 as the fluid 4 is subjected to both acoustic streaming from the acoustic standing wave 18 and thermal flow from the thermal gradient provided by the heating devices 22a and 22b.

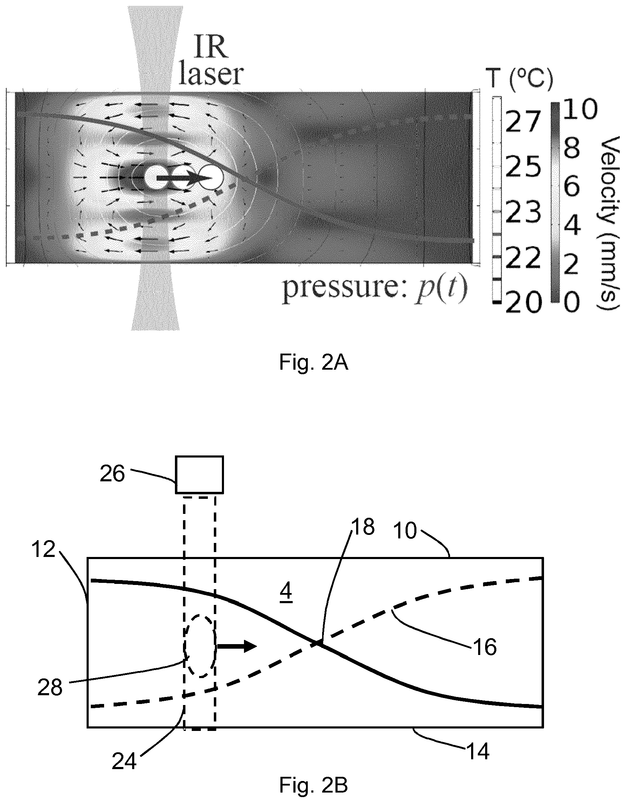

Fig. 2A shows a simulation of the velocity of fluid under the influence of both a standing wave (acoustic velocity field) and a local thermal inhomogeneity. The local thermal inhomogeneity is caused by IR laser radiation irradiating a local portion of the fluid. The fluid that is locally heated obtains, due to its higher temperature, a higher acoustic contrast in relation to the surrounding, unheated fluid. This locally heated fluid will therefore be experience a radial force from the acoustic standing wave which will cause the locally heated fluid to rapidly move towards the pressure node in the center of the channel. The moving locally heated fluid will further entrain any particle. This effect is according to the technology proposed herein useful for manipulating particles that are too small (i.e. sub-micron particles) to travel sufficient distances under the influence of the acoustic standing wave alone to be separated. As an example the concurrent and transient application of the acoustic standing wave and a local thermal inhomogeneity can be used for sorting of particles by being selectively applied when a particle of interest enters the light path of the laser. As the acoustic standing wave and a local thermal inhomogeneity are applied, such as in a short pulse, the fluid around the particle will be heated and rapidly move towards the pressure node, from which the particle can be obtained through a suitably placed outlet. This allows sorting also of small particles as they are entrained by the moving locally heated fluid.

Fig. 2B shows a system for providing an acoustic field in combination with a local thermal inhomogeneity. Here, for clarity, the substrate 2 and ultrasound transducer 20 are not shown. The local thermal inhomogeneity is provided by laser light 24 from a laser 26. In and around the focal point of the laser light 24 locally heated fluid 28 rapidly, upon application of the laser light 24, is heated to a temperature above the temperature of the fluid 4 in the remainder of the channel 10. This locally heated fluid 28 then rapidly is forced towards the pressure node 18 of the acoustic standing wave 16 as indicated by the solid arrow.

As an alternative to applying the laser radiation 24 at a position away from the pressure node 18, the laser radiation can be applied at the pressure node 18. This results in the locally heated fluid 28 being provided at the pressure node 18. The boundary or interface between the locally heated fluid 28 and the surrounding fluid 4 can then provide a dampening or attenuating effect on acoustic streaming in the channel 10. As the locally heated fluid 28 is continuously held at a temperature above the surrounding fluid 4 it will be continuously forced towards the pressure node 18. This will dampen, attenuate or remove acoustic streaming in the center of the channel 1 in the locally heated fluid 28 and can be used to provide volume, i.e. in the locally heated fluid 28, for separating or handling particles which could otherwise not be separated due to being too small, and thereby move too slow, in relation to the velocity of the acoustic streaming.

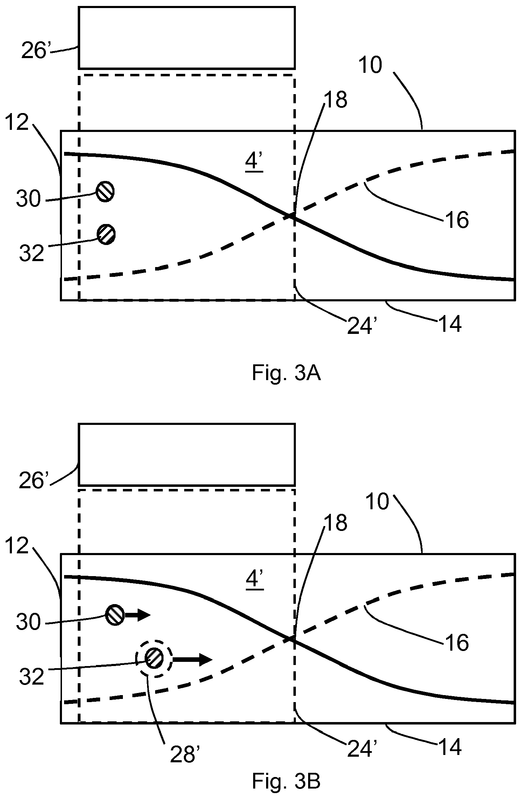

Fig. 3A-B shows a system for providing an acoustic field in combination with a local thermal inhomogeneity spatially located to a particle absorbing wide-field laser radiation. Here wide field laser radiation 24' is provided from a wide field laser 26'. The wide field laser radiation 24' irradiates a large proportion of the cross section of the channel 10 including first and second particles 30 and 32. The fluid 4' may be the same as fluid 4 in fig 2B, however in any case the second particle 32 absorbs more of the wide field laser radiation 24' than the fluid 4' does, while the first particle 30 absorbs less of the wide field laser radiation 24' than the second particle 32.
Fig. 3A shows the situation at the moment the acoustic standing wave 16 and the wide field laser radiation 24' are energized. Fig. 3B shows the situation shortly thereafter. Whereas the fluid 4' absorbs little or none of the wide field laser radiation 24', the second particle 33 significantly absorbs the laser light and is heated so that its temperature increases. This leads to local heating of the fluid 28' surrounding the second particle 32. The second particle 32 together with the heated fluid 28' surrounding the particle 32 are thus rapidly forced towards the pressure node 18, due to the heated fluid 28' surrounding the second particle 32 having a positive acoustic contrast relative to the surrounding non-heated fluid, at a velocity that is significantly higher than that of the first particle 30 which, due to not significantly absorbing the wide field laser radiation 24', is not able to locally heat the fluid 4' surrounding it. Thus the force on the first particle 30 from the acoustic standing wave is limited to that arising from first particle 30, whereas the force on the second particle 32 is higher due to the second particle 32, together with its surrounding heated fluid 28', representing a larger volume thus experiencing a larger force from the acoustic standing wave 16. In addition the surrounding heated fluid 28' has a lower viscosity, due to its higher temperature, than the viscosity of the fluid 4' surrounding the first particle 30, which further lowers the drag on the second particle 32 and its surrounding heated fluid 28' as they move towards the pressure node 18.
By selecting a suitable fluid 4' and using radiation of a wavelength a desired particle that is to be moved absorbs more than other particles, the desired particle can be moved, and thus separated, from other particles which the desired particle could not be separated using the acoustic standing wave 16 alone.
Although the figures show a standing acoustic wave 16 also a traveling acoustic wave has a pressure node, which however moves across the channel 10.

### Feasible modifications

The technology proposed herein is not limited only to the embodiments described above and shown in the drawings, which primarily have an illustrative and exemplifying purpose. This patent application is intended to cover all adjustments and variants of the preferred embodiments described herein, thus the present invention is defined by the wording of the appended claims and the equivalents thereof. Thus, the equipment may be modified in all kinds of ways within the scope of the appended claims.

For instance, it shall be pointed out that structural aspects of embodiments of the method according to the first aspect of the technology proposed herein shall be considered to be applicable to embodiments of the system according to the second aspect of the technology proposed herein, and conversely, methodical aspects of embodiments of the system according to the second aspect of the technology proposed herein shall be considered to be applicable to embodiments of the method according to the first aspect of the technology proposed herein.

It shall also be pointed out that all information about/concerning terms such as above, under, upper, lower, etc., shall be interpreted/read having the equipment oriented according to the figures, having the drawings oriented such that the references can be properly read. Thus, such terms only indicates mutual relations in the shown embodiments, which relations may be changed if the inventive equipment is provided with another structure/design.

It shall also be pointed out that even thus it is not explicitly stated that features from a specific embodiment may be combined with features from another embodiment, the combination shall be considered obvious, if the combination is possible.
Throughout this specification and the claims which follows, unless the context requires otherwise, the word "comprise", and variations such as "comprises" or "comprising", will be understood to imply the inclusion of a stated integer or steps or group of integers or steps but not the exclusion of any other integer or step or group of integers or steps.

## Claims

1. A method of performing an acoustophoretic operation comprising the steps of:
v. providing a fluid,
vi. positioning the fluid in a microfluidic cavity,
vii. subjecting at least one portion of the fluid, in the microfluidic cavity, to an acoustic wave, and
viii. providing, in at least one first region of the at least one portion, a thermal inhomogeneity whereby the temperature of the fluid in the at least one first region differs from the temperature of the fluid in at least one second region of the remainder of the at least one portion.

2. The method according to claim 1, wherein the temperature of the fluid in the at least one first region differs by at least 0.1°C from the temperature of the fluid in the at least one second region of the at least one portion.

3. The method according to any of the preceding claims, wherein the thermal inhomogeneity comprises a thermal gradient throughout at least the at least one first region.

4. The method according to any of the preceding claims, wherein the thermal inhomogeneity is effected by directing electromagnetic radiation into the at least one first region.

5. The method according to claim 4, wherein the wavelength of the electromagnetic radiation is configured to heat the fluid by absorption.

6. The method according to claim 4, wherein the fluid comprises a particle or molecule and the wavelength of the electromagnetic radiation is configured to heat the particle or molecule by absorption, and wherein the absorption coefficient of the particle or molecule for the electromagnetic radiation differs from the absorption coefficient of the fluid for the electromagnetic radiation.

7. The method according to any of claims 4-6, wherein the wavelength of the electromagnetic radiation comprises I R-light and visible light.

8. The method according to any preceding claim, wherein the acoustic wave is an acoustic standing wave.

9. A microfluidic system for performing an acoustophoretic operation, the system comprising:
a substrate with a microfluidic cavity formed in the substrate, the microfluidic cavity having an inlet for allowing a fluid into the microfluidic cavity,
an ultrasound transducer connected to the substrate for generating an acoustic wave in at least one portion of the fluid in the microfluidic cavity,
a drive circuit connected to the ultrasound transducer and configured to drive the ultrasound transducer to provide the acoustic wave, and
a thermal device configured to provide, in at least one first region of the at least one portion, a thermal inhomogeneity whereby the temperature of the fluid in the at least one first region differs from the temperature of the fluid in at least one second region of the remainder of the at least one portion.

10. The microfluidic system according to claim 9, wherein the thermal device comprises a LED and/or laser arranged for irradiating the at least one first region of the at least one portion.

11. The microfluidic system according to any of the claims 9-10, wherein the thermal device further comprises a heating device for heating the substrate for causing a thermal gradient throughout the substrate and throughout the microfluidic cavity.

12. The microfluidic system according to any of the claims 9-11, wherein the thermal device further comprises a heating device positioned within the microfluidic cavity or on an inner wall of the microfluidic cavity for heating the fluid in the microfluidic cavity.

13. The microfluidic system according to any of the claims 9-12, further comprising
a detector configured for detecting that a particle of interest is present in the at least one first region and for outputting a signal when the particle of interest is present in the at least one first region, and
a relay device configured for receiving the signal from the detector and for energizing the drive circuit and the thermal device for causing movement of the fluid in the at least one first region and the particle of interest.

14. The microfluidic system according to any of the claims 9-13, wherein the acoustic wave is an acoustic standing wave.

15. Use of a thermal inhomogeneity provided in at least one first region of at least one portion of a fluid a microfluidic cavity in combination with an acoustic wave provided in the fluid for performing an acoustophoretic operation in or on the fluid.
